(19) Europäisches Patentamt
European Patent Office
Office européen des brevets

(11) **EP 3 658 093 B1**

(12) **EUROPEAN PATENT SPECIFICATION**

(45) Date of publication and mention
of the grant of the patent:
**09.04.2025 Bulletin 2025/15**

(21) Application number: **17918863.6**

(22) Date of filing: **27.07.2017**

(51) International Patent Classification (IPC):
*A61F 13/15* (2006.01)    *A61F 13/49* (2006.01)

(52) Cooperative Patent Classification (CPC):
**A61F 13/49011; A61F 13/15203**

(86) International application number:
**PCT/CN2017/094631**

(87) International publication number:
**WO 2019/019081 (31.01.2019 Gazette 2019/05)**

(54) **PANT-TYPE ABSORBENT ARTICLE**

HOSENÄHNLICHER SAUGFÄHIGER ARTIKEL

ARTICLE ABSORBANT DE TYPE CULOTTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**03.06.2020 Bulletin 2020/23**

(73) Proprietor: **The Procter & Gamble Company
Cincinnati, OH 45202 (US)**

(72) Inventors:
• **CHENG, Chunmin
Beijing 101312 (CN)**
• **LIU, Hui
Beijing 101312 (CN)**
• **MORIMOTO, Koichi
Beijing 101312 (CN)**
• **TONG, Ling
Beijing 101312 (CN)**

(74) Representative: **P&G Patent Germany
Procter & Gamble Service GmbH
Sulzbacher Straße 40
65824 Schwalbach am Taunus (DE)**

(56) References cited:
EP-A1- 2 517 681          EP-A1- 2 517 681
WO-A1-2013/180612      WO-A1-2016/029370
WO-A1-2016/029370      WO-A1-2016/101195
WO-A1-2016/101195      CN-U- 206 304 050
JP-A- 2007 195 647        US-A1- 2014 144 579
US-A1- 2017 165 128

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to pant-type absorbent articles having an elastic belt having zones of particular stiffness profiles.

BACKGROUND OF THE INVENTION

Infants and other incontinent individuals wear absorbent articles such as diapers to receive and contain urine and other body exudates. Pull-on absorbent articles, or pant-type absorbent articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult, and also for younger babies requiring a soft fit around the waist opening and leg openings.
Pant-type articles may take various structures wherein the circumference of the waist opening and vicinity thereof is made elastic enough to facilitate the wearer or the caregiver to expand the article and insert the wearer's legs into the leg openings for wearing the article. The region of the waist circumference and vicinity thereof is often referred to as the elastic belt. One type of structure for the pant-type article is the belt-type pant having a central chassis to cover the crotch region of the wearer and a separate elastic belt defining the waist opening and leg opening, such as described in PCT Publication WO 2006/17718A. Another type of structure for the pant-type article is the uni-body pant configured such that the outer cover of the article completely covers the entirety of the garment-facing surface of the article, wherein the portion configured to stretch about the torso is considered the elastic belt region.
Whatever the structure of the pant-type article may be, these articles are typically so configured to accommodate size and configuration ranges by providing the elastic belt region very stretchable and comfortable to wear, yet with reliable fit such that sufficient protection against sagging and leakage may be provided. Further, the elastic belt region may be provided elastic by many elastic strands running in the transverse direction of the article. For those wearers having a proportionally big front waist/belly compared to the back waist, which includes a majority of young children under 36 months old, the front elastic belt region has a tendency to sag or fold during wear, due to the pressure from the front waist. Such sagging or folding provides a negative impression of fit and protection, and may also be uncomfortable for the wearer. However, simply providing this region with higher tensile stress would cause further discomfort for the wearer.
Based on the foregoing, there is a need for an absorbent article providing improved fit properties of the front elastic region, and more specifically, to prevent sagging and folding of the front elastic region. There is also a need for providing such an absorbent article economically.
WO 2016/029370 relates to a wearable article comprising a main body and a ring-like elastic belt with a front belt and a back belt. The entirety of the length of the belt side edge of the front belt is seamed with a certain length of the belt side edge of the back belt to define a seam length. The front and back belts are each divided into 4 zones extending in the transverse direction and defined by its location from the distal edge to the proximal edge relative to the percentage of the seam length. The tensile stress of the front leg zone is no more than 50% of the tensile stress of the front proximal tummy zone, and the tensile stress of the back leg zone is no more than 100% of the tensile stress of the back proximal tummy zone.
EP 2 517 681 discloses a wearing article having a chassis bent in a crotch region. A diaper is folded along an imaginary lateral center line so that front and rear waist regions are in contact with each other. In such a folded state, first folds extending in the longitudinal direction are defined in portions of gasket cuffs which are biased toward the front waist region relative to the imaginary lateral center line. Second folds which extend in the longitudinal direction Y are formed in containment cuffs.
WO 2016/101195 relates to an array of wearable articles comprising at least first, second, and third articles. Each article comprises a main body and a ring-like elastic belt. The ring-like elastic belt comprises a front belt and a back belt. At least the front belt or the back belt is an elastic member having an elastic member width in the transverse direction and a non-elastic region.

SUMMARY OF THE INVENTION

The present invention is directed to an absorbent article as defined by claim 1, the article being continuous in a longitudinal direction and a transverse direction comprising a front elastic belt region, a back elastic belt region, a crotch region, a waist opening and two leg openings; the article further comprising a center chassis comprising an absorbent core which extends longitudinally from the front elastic belt region to the back elastic belt region;

the front elastic belt region having a front absorbent core stiffness edge wherein the distance from the distal edge to the front absorbent core stiffness edge defines a length L2, wherein L2 is not greater than about 77mm, wherein the front elastic belt region is divided into 3 zones extending in the transverse direction and defined by its location from the distal edge wherein; 0-50% of L2 is a Zone 1, 50-100% of L2 is a Zone 2, and the remainder of the front elastic belt region is a Zone 3;

wherein Zone 1 has a Vertical Stiffness Z1, Zone 2 has a Vertical Stiffness of Z2, and Zone 3 has a Vertical Stiffness of Z3 according to the measurements herein, and having a relationship of $Z1 \leq Z2 \leq Z3$;

wherein the article having a Stretch Circumference Force and a Full Circumference according to the measurements herein, wherein the ratio of Stretch Circumference Force to Full Circumference is less than about 0.01 (N/mm).

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

Figure 1 is a perspective view of one embodiment of an absorbent article of the present invention.
Figure 2 is a schematic plan view of one embodiment of an absorbent article of the present invention with the seams unjoined and in a flat uncontracted condition showing the garment facing surface.
Figure 3 is a schematic side plan view of embodiments of wearable articles of the present invention in a flat uncontracted condition showing the garment facing surface.
Figures 4A-4C are schematic views of an example of a plunger, a stage, and a sample fixture according to the "Vertical Stiffness Measurement" herein.
Figure 5 is a schematic view of an example of a hanger-type sample holding fixture according to the "Whole Article Force Measurement" herein.

DEFINITIONS

As used herein, the following terms shall have the meaning specified thereafter:
"Absorbent article" refers to articles of wear which may be in the form of pants, taped diapers, incontinent briefs, feminine hygiene garments, and the like. The "absorbent article" may be so configured to also absorb and contain various exudates such as urine, feces, and menses discharged from the body. The "absorbent article" may serve as an outer cover adaptable to be joined with a separable disposable absorbent insert for providing absorbent and containment function, such as those disclosed in PCT publication WO 2011/087503A.
"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".
"Longitudinal" refers to a direction running substantially perpendicular from a waist edge to an opposing waist edge of the article and generally parallel to the maximum linear dimension of the article.
"Transverse" refers to a direction perpendicular to the longitudinal direction.
"Proximal" and "distal" refer respectively to the position closer or farther relative to the longitudinal center of the article.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).
"Disposed" refers to an element being located in a particular place or position.
"Joined" refers to configurations whereby an element is directly secured to another element by affixing the element directly to the other element and to configurations whereby an element is indirectly secured to another element by affixing the element to intermediate member(s) which in turn are affixed to the other element.
"Film" refers to a sheet-like material wherein the length and width of the material far exceed the thickness of the material. Typically, films have a thickness of about 0.5 mm or less.
"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to 0 ± 2, and a b* value equal to 0 ± 2 according to the CIE L* a* b* color system.


DETAILED DESCRIPTION OF THE INVENTION

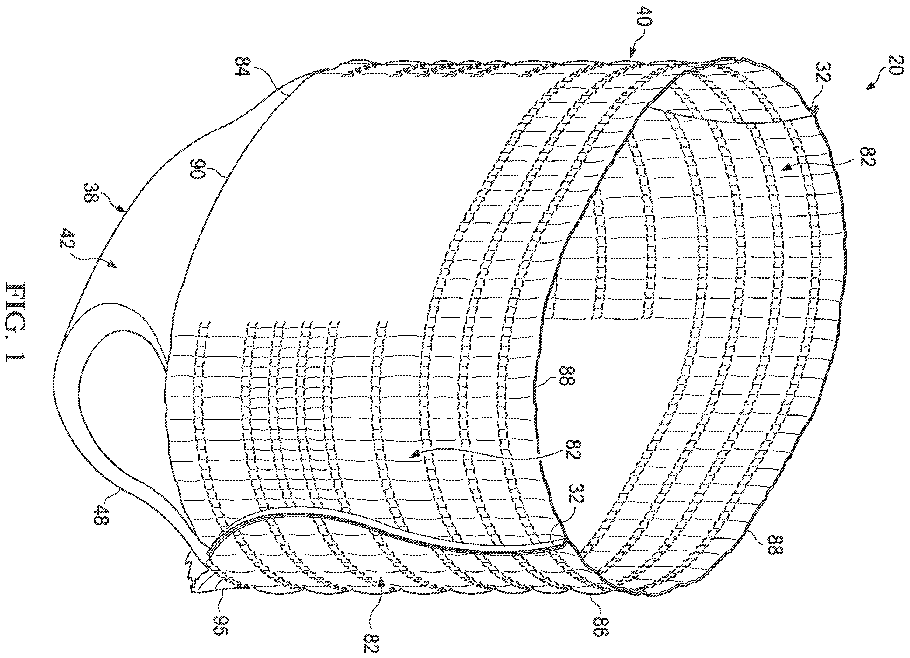

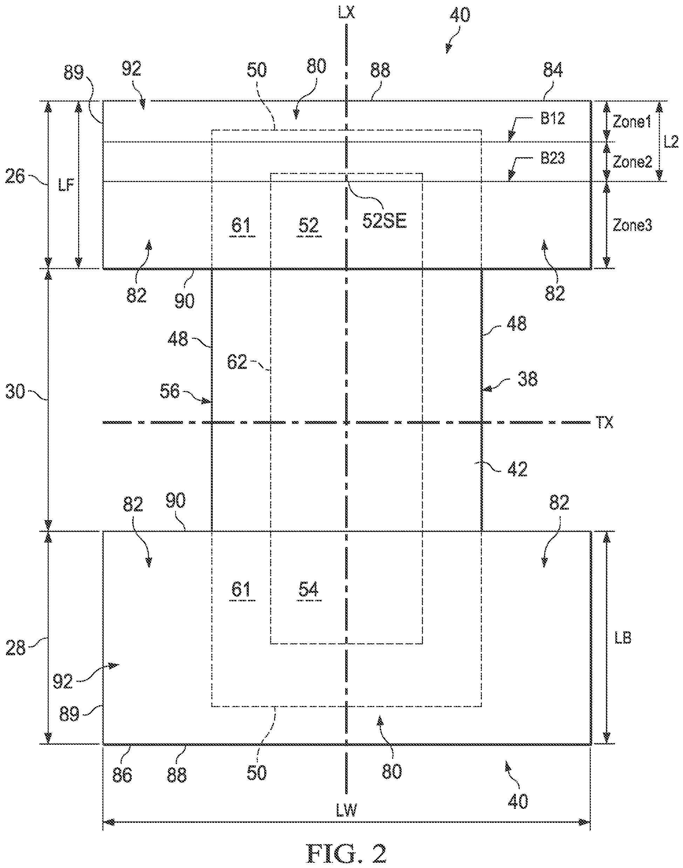

Figure 1 is a perspective view of an embodiment of the absorbent article 20 of the present invention and Figure 2 is a schematic plan view of the same article with the seams unjoined and in its flat uncontracted condition showing the garment-facing surface. The absorbent article 20 has a longitudinal centerline LX which also serves as the longitudinal axis, and a transverse centerline TX which also serves as the transverse axis. The absorbent article 20 has a body facing surface, a garment facing surface, a front region 26, a back region 28, a crotch region 30, and side seams 32 which join the front region 26 and the back region 28 to form two leg openings and a waist opening.

The absorbent article 20 is a belt-type pant comprising a central chassis 38 to cover the crotch region of the wearer, a front belt 84 and a back belt 86 (hereinafter may be referred to as "front and back belts"), the front and back belts 84, 86 forming a discrete ring-like elastic belt 40 (hereinafter may be referred to as "waist belt") extending transversely defining the waist opening. For the belt-type pant, the discrete ring-like elastic belt 40 may also be referred to as the elastic belt region 40. For the belt-type pant, the front and back belts 84, 86 and the central chassis 38 jointly define the leg openings. The absorbent article 20 may be a uni-body type pant wherein the central chassis 38 is continuous with the front and back belt 84, 86, wherein the leg openings are continuously formed. For the uni-body pant, the belt portion existing between the side seams are considered the elastic belt region 40, wherein the elastic belt region 40 is considered to terminate by an imaginary line running in the transverse direction between the proximal edges of the side seams. The remainder of the article except the elastic belt region 40 is considered the crotch region 30.

The central chassis 38 comprises a topsheet, a backsheet and an absorbent core 62 disposed between the topsheet and the backsheet, and may further comprise an outer cover layer 42 for covering the garment-facing side of the backsheet. The topsheet may be a water permeable substrate. The backsheet may be a water impermeable film. The outer cover layer 42 may be a nonwoven sheet. The central chassis 38 contains an absorbent core 62 for absorbing and containing body exudates disposed on the central chassis 38, and may comprise an absorbent material non-existing region 61 surrounding the periphery of the absorbent core 62. The absorbent material non-existing region 61 may be made of the topsheet and/or the backsheet and/or the outer cover layer 42 and/or other parts configuring the central chassis 38. In the embodiment shown in Figure 2, the central chassis 38 has a generally rectangular shape, left and right longitudinally extending side edges 48 (hereinafter may be referred to as "side edge") and front and back transversely extending end edges 50 (hereinafter may be referred to as "end edge"). The absorbent core 62 may exist through the entire longitudinal dimension of the crotch region and extending at least partly in the front elastic belt region 84; or at least partly in both the front and back elastic belt region. The central chassis 38 has a front waist panel 52 positioned in the front region 26 of the absorbent article 20, a back waist panel 54 positioned in the back region 28, and a crotch panel 56 between the front and back waist panels 52, 54 in the crotch region 30. The center of the front belt 84 is joined to a front waist panel 52 of the central chassis 38, the center of the back belt 86 is joined to a back waist panel 54 of the central chassis 38, the front and back belts 84, 86 each having a left side panel and a right side panel 82 where the central chassis 38 does not overlap. The central chassis has a crotch panel 56 positioned between the front waist panel 52 and the back waist panel 54. For the belt-type pant, the front and back belt 84, 86 may be discontinuous of each other in the longitudinal direction.

The absorbent core 62 includes an absorbent layer and an acquisition layer. The absorbent layer is the region wherein absorbent materials having a high retention capacity, such as superabsorbent polymers, are present. The absorbent layer is substantially cellulose free. Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multi constituent fibers such as side by side, core/sheath or island in the sea type fibers. Such synthetic fibers may be formed via a spunbonding process or a meltblowing process. The acquisition layer facilitates the acquisition and the distribution of body exudates and may be placed between the topsheet and the absorbent layer. The acquisition layer may include cellulosic fibers.

Referring to Figure 2, the absorbent core 62 includes a front absorbent core stiffness edge 52SE disposed on the front waist panel 52. The front absorbent core stiffness edge 52SE is a point along the longitudinal centerline LX where the absorbent core 62 takes a significant change in caliper. For absorbent core structures wherein the end edge of the absorbent layer and the end edge of the acquisition layer are matched, such matched end edge is the front absorbent core stiffness edge 52SE. For absorbent core structures wherein the end edge of the absorbent layer and the end edge of the acquisition layer do not match, the either end edge effecting a greater change in caliper is considered the front absorbent core stiffness edge 52SE. Thus, the front absorbent core stiffness edge 52SE may exist within the absorbent core 62, as in Figure 2. When the end edge of caliper change takes a contour that is not parallel with the transverse axis TX, the point matching the longitudinal centerline LX is the front absorbent core stiffness edge 52SE.

The absorbent layers may be disposed in plurality in the absorbent core 62. Some portions of the absorbent layers may be configured to have substantially no absorbent material to form a channel or a plurality of channels. Channels may be useful for allowing the absorbent core to bend upon swelling with fluids, such that the absorbent article conforms to the wearer's body after swelling and prevent sagging of the article. The channels may also be formed in the acquisition layer, and may be configured to at least partly match the channels of the absorbent layer in the thickness direction.

The elastic belt region 40 of the article of the present invention acts to dynamically create fitment forces and to distribute the forces dynamically generated during wear. The front and back elastic belt regions 84, 86 may be joined with each other only at the side edges 89 at the seams 32 to form a wearable article having a waist opening and two leg openings. Each leg opening may be provided with elasticity around the perimeter of the leg opening. The elasticity around the leg opening may be provided by the combination of elasticity from the front elastic belt region 84, the back elastic belt region 86, and any from the central chassis 38.

The transverse width of the backsheet and the outer cover layer 42 may be the same, or may be varied (not shown). For example, the backsheet may have a shorter transverse width compared to that of the outer cover layer 42. By such configuration, the longitudinal side edges 48 of the crotch region 30, which make part of the leg openings, may have better breathability. Further, such configuration may provide cost saving.

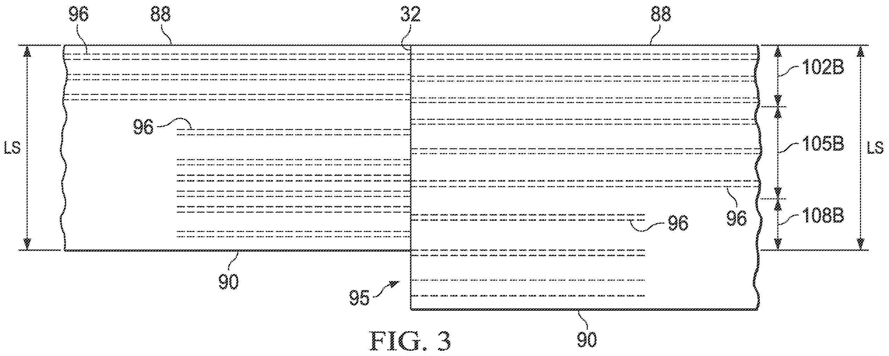

Figure 3 observes both the front elastic belt region 84 and back elastic belt region 86 from the side seam. Referring to Figures 2 and 3, the front elastic belt region 84 and the back elastic belt region 86 are each formed by a laminate comprising a plurality of elastic bodies 96 running in the transverse direction, an inner sheet 94, an outer sheet 92, and the laminate may further comprise an outer sheet fold over (not shown) wherein the outer sheet fold over is an extension of the outer sheet material formed by folding the outer sheet material at the distal edge 88 of the front and back belts; wherein the belt elastic bodies 96 are sandwiched between two of these sheets. The front elastic belt region 84 and the back elastic belt region 86 may each be made only by elastic bodies 96, the inner sheet 94, the outer sheet 92, and the outer sheet fold over. The belt elastic bodies 96 extend in the transverse direction to provide a ring like elastic belt 40 when the front elastic belt region 84 and the back elastic belt region 86 are joined. All of the elastic bodies 96 may extend in the transverse direction substantially parallel to each other. Such an article may be economically made. The front and back elastic belt regions 84, 86 each have transversely continuous proximal and distal edges, the proximal edge 90 being located closer than the distal edge 88 relative to the longitudinal center of the article. The elastic bodies 96 may be disposed in the same or different denier, interval, and force between the front and back, as well as in different longitudinal positions of the belt.

Still referring to Figures 2 and 3, the front and/or back elastic belt regions 84, 86 may be treated such that certain of the area overlapping the front and/or back waist panel 52, 54 of the central chassis 38 are removed of elasticity. Removal of elasticity from a certain area of the front and/or back waist panel 52, 54 may be advantageous in that elasticity in the front and/or back area overlapping the absorbent core 62 may cause bunching of the absorbent layer or any of the layers in the absorbent core 62 and interfere with close fit of the central chassis 38 to the wearer. At least a portion of, or at least 10% of, or at least 20% of, or at least 30% of, the elasticity of; at least one of, or at least half of, or at least two thirds of, the elastic bodies are removed in the region overlapping with the front and back waist panels 52, 54 of the central chassis 38. The elastic bodies 96 overlapping the absorbent material non-existing region 61 may be disposed in active elasticity for good fit of the central chassis 38. This may be advantageous in preventing leakage. The entire area where the elastic bodies 96 overlap with the absorbent core 62 may be removed of its elasticity. Alternatively, the elastic bodies 96 toward the distal edges of the absorbent core 62 may be disposed in active elasticity.

Referring to Figure 2, when assembled as an article, the front absorbent core stiffness edge 52SE as mentioned above is disposed on the front elastic belt region 84, wherein the distance from the waist opening to the front absorbent core stiffness edge 52SE defines a length L2. L2 is no greater than about 77mm, or may be no greater than about 74mm. Referring to L2, the front elastic belt region is divided into 3 zones extending in the transverse direction and defined by its location from the distal edge 88 wherein; 0-50% of L2 is a Zone 1, 50-100% of L2 is a Zone 2, and the remainder of the front elastic belt region is a Zone 3, wherein each zone may have a Vertical Stiffness according to the Vertical Stiffnesss Measurement herein. What is meant by Vertical Stiffness is the stiffness peak force (gf) required to compress the zones of the front elastic belt region 84 in the direction of the longitudinal axis LX, which is believed to simulate the stiffness exerted by the article in resistance to the pressure to deform the article in the vertical direction as worn on the wearer. Zone 1 may be disposed of elastic bodies 96 continuous through the transverse length of the front elastic belt region 84. All of the elastic bodies 96 disposed in Zone 1 may be continuous. Zone 2 may be disposed of elastic bodies 96 continuous through the transverse length of the front elastic belt region 84. All of the elastic bodies 96 disposed in Zone 2 may be continuous, or some of the elastic bodies 96 may be deprived of elasticity in the transverse center of the zone. In light of the proportion of the young child, Zone 2 may match the protruded front waist under the navel. On the other hand, Zone 3 may be disposed of elastic bodies 96 which are deprived of elasticity in the transverse center of the zone. All of the elastic bodies 96 disposed in Zone 3 may be deprived of elasticity in the transverse center of the zone, or some may be continuous. When Zone 1 has a Vertical Stiffness of Z1, Zone 2 has a Vertical Stiffness of Z2, and Zone 3 has a Vertical Stiffness of Z3 according to the measurements herein, they have a relationship of Z1≤Z2≤Z3. Namely the Vertical Stiffness Z2 of Zone 2 is not lower than the Vertical Stiffness Z1 of Zone 1, and the Vertical Stiffness Z3 of Zone 3 is not lower than the Vertical Stiffness Z2 of Zone 2. Without being bound by theory, by having such profile of Vertical Stiffness from the waist opening towards the center of the crotch region, sagging and folding of the front elastic belt region 84 may be prevented.

The aforementioned Zones 1, 2, and 3 may be imparted Vertical Stiffness by a number of measures including, but not limited thereof, 1) the number of layers of materials, 2) the stiffness of the materials, 3) the force provided by the disposed elastic bodies 96, and 4) method of bonding the materials, for example, by adhesive. In that Zone 3 comprises the absorbent core, this zone has the most number of layers of materials, as well as stiffness imparted by the absorbent core. Thus, Z3 may easily be much greater than Z1 and Z2. Meanwhile, in that Zone 2 is in the vicinity of the edge of the absorbent core 62, there may be desire to avoid having continuous elastic bodies 96 in this zone. Thus, even when the number of layers of material of Zone 2 may be more than that of Zone 1, Z2 may be smaller than Z1 due to lack of force of the elastic bodies 96. Without being bound by theory, it is believed that when Z2 is lower than both Z1 and Z3, the pressure coming from the protruded front waist of the wearer corresponding to Zone 2 may force Zone 2 to fold, and even lead to sagging of the entire front waist elastic region 84. It is further believed that, when Z2 is lower than both Z1 and Z3, the greater the gradient between Z2 compared to Z1 and Z3, the greater the tendency of folding and sagging. On the other hand, there is interest to control the stiffness of Zone 2 from becoming too high that it may cause unnecessary discomfort to the wearer. Thus, the Vertical Stiffness of Zones 1, 2, and 3 is adjusted to effect a relationship of Z1≤Z2≤Z3. Z3, and hence all of Z1-Z3, may be less than 65gf.

Further, in order to control the tightness perception or softness perception of the article, the following force parameters may be used as guidance. The article of the present invention has a suitable Stretch Circumference Force (N) of less than 7N according to the Whole Article Force Measurement below. What is meant by Stretch Circumference Force is the loading force at a certain stretch level which is believed to simulate initial stretch experience felt by the user when inserting hands and stretch opening the article. The level of stretch which is believed to be felt by the user when stretch opening the article is represented by the 70% Stretch Circumference (mm) also according to the Whole Article Force Measurement herein. The article of the present invention has a Stretch Circumference Force of no greater than about 7N, or no greater than about 6.5N, or no greater than about 6N. By having such Stretch Circumference Force, the elastic belt can be easily opened and applied. Without being bound by theory, it is believed that the lower the Stretch Circumference Force, the elastic belt region may be stretched with less force, thus less tight and softer the perception of the elastic belt region by the user.

The article of the present invention may have a Full Circumference (mm) according to the Whole Article Force Measurement below. The Full Circumference means to measure the maximum circumference to which the article may be stretch opened. The maximum circumference at 19.6N is defined as the Full Circumference (mm). In the present invention, the ratio of Stretch Circumference Force to Full Circumference is less than about 0.01 (N/mm). Without being bound by theory, it is believed that the smaller the ratio, the softer fit may be provided for the same material used for forming the transverse dimension of the elastic belt region.

The article of the present invention has a suitable Fit Circumference Force (N) of no more than 2N according to the Whole Article Force Measurement below. What is meant by Fit Circumference Force is the unloading force at a certain stretch level which is believed to simulate the force felt by the wearer while wearing the article. The level of stretch which is believed to be felt by the wearer while wearing the article is also represented by the 70% Stretch Circumference (mm). The article of the present invention has a Fit Circumference Force of no less than about 2N. By having such Fit Circumference Force, the elastic belt provides good fit to prevent sagging and leakage. Without being bound by theory, it is believed that by having a relatively low Stretch Circumference Force of no greater than about 7N in combination with a minimum Fit Circumference Force of no less than about 2N, an elastic belt region having ease of application and a secure yet soft fit may be provided. Without being bound by theory, it is believed that by providing the aforementioned Stretch Circumference Force and Fit Circumference Force, the pant-type article of the present invention provides an overall satisfactory tactile sense to the user upon touching, applying, and wearing the article.

Referring to Figure 3, the tensile stress (N/m) of the front and back elastic belts 84, 86, respectively, may be profiled in order to provide the benefits of the present invention. The tensile stress may be measured, for example, by the Belt Zone Tensile Stress Measurement described herein below. When the elasticity of the front and back elastic belts 84, 86 are provided by a plurality of elastic bodies 96 running in the transverse direction, the tensile stress may be adjusted by one or more of the following methods; 1) elongation rate of the elastic body 96; 2) density (dtex) of the elastic body 96; 3) longitudinal interval of multiple elastic bodies 96; and 4) effective length of elasticity of the elastic body 96 in the transverse direction. The elastic bodies may be elastic strands having a dtex of from about 310 to about 1520 and disposed at an elongation of from about 110% to about 290%. By elongation, "0% elongation" is meant the original length of the elastic body 96.

Some elastics may be disposed to impart higher tensile stress in certain regions. A plurality of elastics may collectively be disposed to impart a relatively higher or lower tensile stress compared to other regions. Such collective elastics are also referred to herein as zones. The zones of the front elastic belt region 84 are defined above as Zones 1, 2, and 3. The zones of the back elastic belt region 86 may also extend in the transverse direction and defined by its location from the distal edge 88 to the proximal edge 90 relative to the percentage of the seam length LS, as such:

|      | Percentage from distal edge | Zone definition |
|------|-----------------------------|-----------------|
| Back | 0-30%                       | Waist zone 102B |
|      | 30%-75%                     | Tummy zone 105B |
|      | 75%-100%                    | Leg zone 108B   |

Zones of higher tensile stress may be disposed with an array of 2-4 elastic strands having an interval within the array of between 2-4mm. The tensile stress of Zone 3 may be higher than any of Zones 1 and 2. The tensile stress of the back tummy zone 105B may be higher than the tensile stress of any other zone. The array may be disposed on the back tummy zone 105B. The array may be disposed on Zone 3.

1. Vertical Stiffness Measurement

This test method is used to measure Vertical Stiffness which is the stiffness peak force (gf) required to compress the front elastic belt region 84 of an absorbent article along the longitudinal axis LX in a vertical direction of the article. A finished article is used with its seams at least partially left attached. Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at $23 \pm 2\,°C$ and $50 \pm 5\,\%$ relative humidity. Samples are pre-conditioned in a room maintained at $23 \pm 2\,°C$ and $50 \pm 5\,\%$ relative humidity, for at least 2 hours prior to testing. Samples are used as immediately taken out from a package with no treatment.

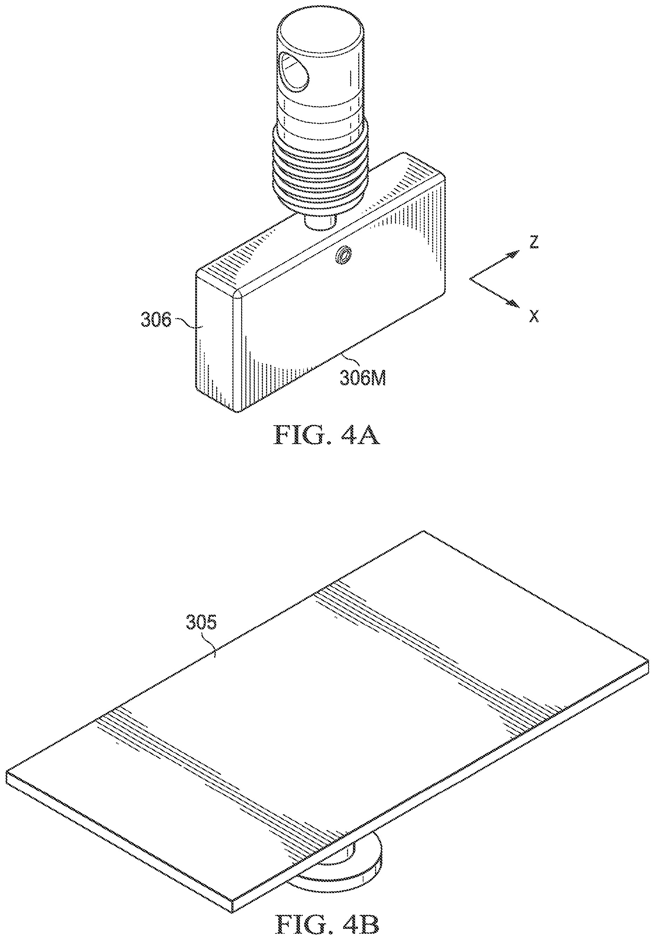

Referring to Figures 4A and 4B, the tensile tester is fitted with a plunger 306 (custom made 50mm width×12mm depth×32mm height) for the upper fixture, and a stage 305 (150mm×310mm) for the lower fixture. The instrument is set up to go through the following steps:

| Crosshead Speed       | 2.0mm/s |
|-----------------------|---------|
| Final extension Point | 10 mm   |
| Hold Time             | 0       |
| Number of Cycles      | 1       |

(continued)

| Data Acquisition Rate | 50Hz |
|---|---|

1) Referring to Figure 2, mark a horizontal line across the entirety of the garment-facing side of the sample along the front absorbent core stiffness edge 52SE. Such line B23 is defined as the Zone 2-3 boarder.

2) Measure the longitudinal length L2 from the distal edge 88. Mark a horizontal line across the entirety of the garment-facing side of the sample at the center point of L2. Such line B12 is defined as the Zone 1-2 boarder. The area above the Zone 1-2 boarder B12 is Zone 1, the area between the Zone 1-2 boarder B12 and Zone 2-3 boarder B23 is Zone 2, and the remainder of the front elastic belt region is Zone 3. Accordingly, Zones 1 - 3 extend in the transverse direction and defined by its location from the distal edge 88 wherein; 0-50% of L2 is Zone 1, 50-100% of L2 is Zone 2, and the remainder of the front elastic belt region is Zone 3.

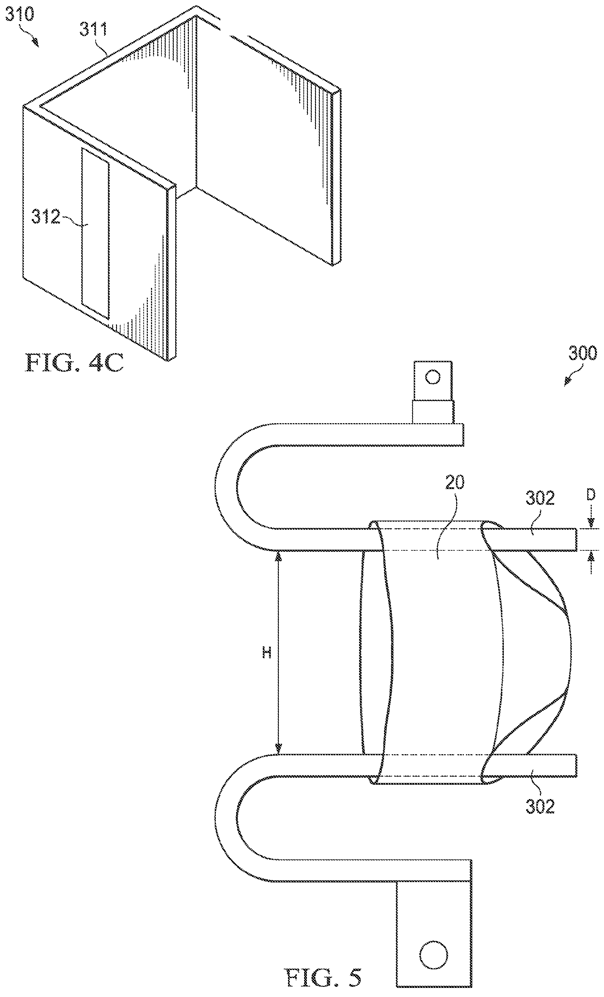

3) Attach the marked sample to the outer periphery of a sample fixture 310 (120mm width×120mm depth×140mm height) as in Figure 4C with the center of the front elastic belt region matching the open plane of the sample fixture 310, and so that the distal edge 88 or waist opening of the elastic belt is matched with the upper edge 311 of the sample fixture 310. Hook material 312 may be provided on the outer walls of the sample fixture 310 to aid secure attachment of the sample.

4) Lay the sample attached to the sample fixture 310 on the MTS stage 305 as in Figure 4B, and align the distal edge 88 or waist opening of the front elastic belt region 84 with the plunger 306 as in Figure 4A by matching the X-axis of the plunger 306 with the transverse axis TX of the sample, and the Z-axis of the plunger 306 with the thickness direction of the sample. The center point 306M of the plunger should match the longitudinal centerline LX of the front belt elastic region 84.

5) The load cell is tared and the crosshead is lowered to make the plunger slightly touch the distal edge 88 or waist opening, ensure the force values shows around 2gf (±0.5gf).

6) The test is initiated and the crosshead moves down at 2.0 mm/s from the distal edge 88 or waist opening in the vertical direction in Zone 1, until an extention of 10mm is attained, then the crosshead immediately returns to the initial position at the same speed. The peak load during the extension segment of the test is recorded as Vertical Stiffness Z1.

7) After Zone 1 measurement, remove the sample from the sample fixture 310. Use scissors to remove Zone 1 from the sample by cutting along the side seams for a length of 0.5L2 and Zone 1-2 boarder B12. The remainder length of the side seams are kept intact. The Zone 1-2 boarder B12 now becomes the waist opening of the front belt elastic region 84.

8) Reattach the once cut sample to the sample fixture 310, with the distal edge 88 of the back elastic belt region 86 matched with the upper edge 311 of the sample fixture 310 and the waist opening of the front elastic belt region 84 existing below the upper edge 311 of the sample fixture 310. Repeat Steps 4) to 6), albeit with Zone 1 replaced with Zone 2. The peak load during the extension segment of the test for Zone 2 is recorded as Vertical Stiffness Z2.

9) After Zone 2 measurement, remove the sample from the sample fixture 310. Use scissors to remove Zone 2 from the sample by cutting along the side seams for another length of 0.5L2 and Zone 2-3 boarder B23. The remainder length of the side seams are kept intact. The Zone 2-3 boarder B23 now becomes the waist opening of the front belt elastic region 84.

10) Reattach the twice cut sample to the sample fixture 310, with the distal edge 88 of the back elastic region matched with the upper edge 311 of the sample fixture 310 and the waist opening of the front elastic belt region 84 existing below the upper edge 311 of the sample fixture 310. Repeat Steps 4) to 6), albeit with Zone 1 replaced with Zone 3. The peak load during the extension segment of the test for Zone 3 is recorded as Vertical Stiffness Z3.

Five samples are analyzed and the average Vertical Stiffness values Z1, Z2, and Z3 are calculated and reported to the nearest 0.1 gf, respectively.

2. Whole Article Force Measurement

Force of a pant-type article is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at 23 ± 2 °C and 50 ± 5 % relative humidity.

The tensile tester is fitted with hanger-type sample holding fixtures 300 as shown in Figure 5. Each fixture comprises a rigid linear rubber-coated horizontal bar section 302 to prevent sample slippage during testing. The outer bar diameter (including the rubber coating) of the horizontal bar sections is 10.0 mm. The central axes of the horizontal bar sections 302 are configured to remain parallel and in the same vertical plane throughout the test procedure. The gauge circumference is determined by the following equation:

$$\text{Gauge Circumference} = 2 \times (H + D + \pi D/2)$$

where H is the vertical gap between the horizontal bar sections 302, and D is the outer diameter of the bar.

The instrument is set up to go through the following steps:

| Crosshead Speed | 254.0mm/min |
|---|---|
| Final Load Point | 19.6 N |
| Hold Time | 0 |
| Number of Cycles | 1 |
| Data Acquisition Rate | 50Hz |

A sample article 20 is inserted onto the upper horizontal bar section 302 so that the bar passes through the waist opening and one leg opening of the article. The crosshead is raised until the specimen hangs above the lower bar and does not touch lower bar 302. The load cell is tared and the crosshead is lowered to enable the lower bar 302 to be inserted through the waist opening and other leg opening without stretching the article. The article is adjusted so that the longitudinal centerline LX of the article is in a horizontal plane halfway between the upper and lower bars 302. The center of the side portion in contact with the bar 302 is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the Initial Gauge Circumference at the same speed. The maximum circumference at 19.6N during the extension segment of the test is recorded.

The maximum circumference at 19.6N is defined as the Full Circumference (mm). The Full Circumference multiplied by 0.7 is defined as the 70% Stretch Circumference (mm). The Stretch Circumference Force is defined as the force at the 70% Stretch Circumference during the load (extension) segment of the test. The Fit Circumference Force is defined as the force at 70% Stretch Circumference during the unload (contraction) segment of the test.

Five samples are analyzed and their average Initial Gauge Circumference, average Full Circumference, average Stretch Circumference Force and average Fit Circumference Force are calculated and reported to the nearest 1 mm or 0.01 N, respectively.

3. Belt Zone Tensile Stress Measurement

The Zone Tensile Stress Measurement herein is for articles wherein the front belt and the back belt form a ring-like elastic belt, and all of the elastic bodies sandwiched between the inner sheet and the outer sheet run in the transverse direction substantially parallel to each other.

The tensile stress (N/m) is calculated by tensile force (N) divided by the longitudinal length (m) of the specimen. Force may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10 and 90% of capacity of the load cell. The instrument is calibrated according to the manufacturer's instructions. All testing is performed in a room maintained at $23 \pm 2\,°C$ and $50 \pm 5\,\%$ relative humidity. The instrument is equipped with single line contact grips at least as wide as the test specimen.

To obtain test specimens, the sample article is opened at both side seams in a manner such that the front and back belts are peeled away from each other without removing the side seam area, and the front and rear elastic belts are removed from the central chassis 38 by separating the bonding between the waist belt and central chassis. Cold spray may be used, paying attention not to make wrinkles in the belt sections. Care is taken not to spray on any belt elastic body 96. The obtained front and back belts 84, 86 are severed into zones Zone 1, Zone 2, Zone 3, 102B, 105B, and 108B as defined above with care not to cut any elastic body 96. Samples are pre-conditioned at $23 \pm 2\,C°$ and $50 \pm 5\%$ relative humidity for two hours prior to testing. Measure the longitudinal length of each specimen by a generic metal ruler to the nearest 1mm. By longitudinal length, what is meant is the dimensions Zone 1, Zone 2, Zone 3, 102B, 105B, and 108B in Figures 2 and 3.

The instrument is set up to go through the following steps. Initial Gauge Length is calculated from the Initial Gauge Circumference which is determined during the Whole Article Force Test using separate identical articles, as described above. The Initial Gauge Length is defined as 50% of the Initial Gauge Circumference. The Final Gauge Length is calculated from the Full Circumference which is determined during the Whole Article Force Test, as described above.

| Crosshead Speed | 254.0 mm/min |
|---|---|
| Data Acquisition Rate | 50Hz |
| Final Gauge Length | 0.5 × Full Circumference |
| Hold Time | 0 |
| Number of Cycles | 1 |

One end of the specimen is clamped into the upper clamp using the side seam area and the load is tared. The other end of the specimen is clamped into the lower clamp also using the side seam area, such that only the side seam areas are behind the contact line of the grip. The test is started and the specimen is extended to the Final Gauge Length at a crosshead speed of 254 mm/min, then immediately returned to the Initial Gauge Length at the same speed. The specimen is extended in the article transverse direction during the test. The unload force at 70% of the Final Gauge Length during the unload segments of the test is recorded.

Five articles are analyzed and the unload forces are recorded for each of the front and back zones 1 Zone 1, Zone 2, Zone 3, 102B, 105B, and 108B. The average tensile force (N) is calculated to the nearest 0.01 N for each zone including the front and back specimens for that zone. The tensile stress for each zone is calculated by the average tensile force (N) divided by the average longitudinal length (m) and reported to the nearest 0.1 N/m.

EXAMPLES

Examples 1 and A-C having the structure of a pant type absorbent article obtained as such are subject to measurements as described above.

Example 1: Size 4 (L-size) belt-type pant article having the configuration of Figure 3 and elastic profile in Table 1 below.

Example A: Size 4 (L-size) belt-type pant article having the configuration of Figure 2 and same dimension as Example 1, with elastic profile in Table 1 below.

Example B: Size 4 (L-size) belt-type pant article sold by the tradename of "Mammy Poko Fashion Girl Pants" with Lot No. 20161104 purchased in China market in 2017.

Example C: Size 4 (L-size) belt-type pant article sold by the tradename of "Goon Premium" with Lot No. 20161217 purchased in China market in 2017.

Table 1

| | Example 1 | Example A |
|---|---|---|
| | Dtex / elongation% / number of elastic bodies | |
| Zone 1 | 540dtex / 160% / 6 | 540dtex / 160% / 6 |
| Zone 2 | 540dtex / 160% / 2 | 540dtex / 160% / 2 tummy cut |
| Zone 3 | 940dtex / 210% / 8 tummy cut<br>540dtex / 160% / 2 tummy cut | 940dtex / 210% / 8 tummy cut<br>540dtex / 160% / 2 tummy cut |
| Waist zone | 540dtex / 160% / 6 | 540dtex / 160% / 6 |
| Tummy zone | 940dtex / 130% / 6 | 940dtex / 130% / 6 |
| Leg zone | 540dtex / 210% / 6 tummy cut | 540dtex / 210% / 6 tummy cut |
| (*1) "tummy cut" in Table 1 refers to removal of elasticity at the central area of elastic strands which overlap the central chassis, resulting in 66% effective length of elasticity. | | |

The stiffness of articles per zone were measured according to the Vertical Stiffness Measurement and Whole Article Force Measurement methods herein with results found in Table 2 below. All examples had a Fit Circumference Force of at least 2N.

Table 2

|  | Example 1 | Example A | Example B | Example C |
|---|---|---|---|---|
| Zone 1 (gf) | 22.8 | 32.2 | 37.2 | 27.4 |
| Zone 2 (gf) | 27.0 | 15.3 | 39.2 | 27.9 |
| Zone 3 (gf) | 62.9 | 56.5 | 70.0 | 36.4 |
| L2 (mm) | 70 | 75 | 55 | 63 |
| Stretch Circumference Force (N) | 6.00 | 5.96 | 7.58 | 6.93 |
| Full Circumference (mm) | 673.8 | 671.6 | 641.3 | 645.2 |
| Ratio of Stretch Circumference force to Full Circumference (N/mm) | 0.0089 | 0.0088 | 0.0118 | 0.0107 |

The article of Example 1 of the present invention provides overall good fit, good fit around the waist of the product, prevents sagging and folding on the front waist, and provides the perception that the front waist is well covered.

## Claims

1. An absorbent article (20) continuous in a longitudinal direction and a transverse direction comprising a front elastic belt region (84), a back elastic belt region (86), a crotch region (30), a waist opening and two leg openings; the article (20) further comprising a center chassis (38) comprising an absorbent core (62) which extends longitudinally from the front elastic belt region (84) to the back elastic belt region (86);

   wherein the central chassis (38) comprises an absorbent core (62) comprising a topsheet, a backsheet and an absorbent core (62) positioned between the topsheet and the backsheet, the absorbent core (62) further comprising an absorbent layer and an acquisition layer, wherein the absorbent layer is substantially cellulose free, wherein the front edge of the absorbent layer is disposed distal from the front edge of the acquisition layer edge, wherein the front edge of the acquisition layer is the front absorbent core stiffness edge (52SE);
   the front and back elastic belt regions (84, 86) each having transversely continuous proximal and distal edges, the proximal edge (90) being located closer than the distal edge (88) relative to the longitudinal center of the article;
   the front elastic belt region (84) having the front absorbent core stiffness edge (52SE) with the distance from the distal edge (88) to the front absorbent core stiffness edge (52SE) defines a length L2, wherein L2 is no greater than 77mm, and wherein the front elastic belt region (84) is divided into 3 zones extending in the transverse direction and defined by its location from the distal edge (88) wherein; 0-50% of L2 is a Zone 1, 50-100% of L2 is a Zone 2, and the remainder of the front elastic belt region (84) is a Zone 3;
   wherein Zone 1 has a Vertical Stiffness of Z1, Zone 2 has a Vertical Stiffness of Z2, and Zone 3 has a Vertical Stiffness of Z3 according to the measurements herein, and having a relationship of $Z1 \leq Z2 \leq Z3$;
   wherein the article (20) having a Stretch Circumference Force and a Full Circumference according to the measurements herein, wherein the ratio of the Stretch Circumference Force to the Full Circumference is less than 0.01 (N/mm),
   wherein the article (20) has a Stretch Circumference Force of less than 7N according to the measurements herein, and
   wherein the article (20) has a Fit Circumference Force or more than 2N according to the measurements herein, and
   wherein the front elastic belt region (84) and the back elastic belt region (86) form a ring-like elastic belt (40), the center of the front elastic belt region (84) is joined to a front waist panel (52) of the center chassis (38), the center of the back elastic belt region (86) is joined to a back waist panel (54) of the center chassis (38), the front and back elastic belt regions (84, 86) each having a left side panel and a right side panel (82) where the central chassis (38) does not overlap, wherein the front and back elastic regions (84, 86) each are a laminate of an inner sheet (94), an outer sheet (92), and a plurality of elastic strands sandwiched therebetween, wherein all of the elastic strands sandwiched between the inner sheet (94) and the outer sheet (92) run in the transverse direction substantially parallel to each other, the entirety of the length of the belt side edge of the front elastic belt region (84) is seamed with a certain length of the belt side edge of the back elastic belt region (86) to define a seam length LS;
   wherein the back elastic belt region (86) divided into 3 zones extending in the transverse direction and defined by its location from the distal edge (88) to the proximal edge (90) relative to the percentage of the seam length LS wherein; 0-30% is a waist zone (102B), 30-75% is a tummy zone (105B), and 75-100% is a leg zone (108B), wherein the tensile stress of the back tummy zone (105B) is higher than the tensile stress of any other zone.

2. The article (20) of Claim 1 wherein Z3 is less than 65gf according to the measurements herein.

3. The article (20) of any of the preceding claims wherein the tensile stress of Zone 3 is higher than the tensile stress of any of Zones 1 or 2.

4. The article (20) of any of the preceding claims wherein the absorbent layer comprises a longitudinally extending channel, the channel being substantially free of absorbent material.

5. The article (20) of any of the preceding claims wherein a channel is further formed in the acquisition layer, wherein the channel in the acquisition layer at least partially matches the channel of the absorbent layer in the thickness direction.

**Patentansprüche**

1. Absorptionsartikel (20), der in einer Längsrichtung und einer Querrichtung ununterbrochen ist, umfassend einen vorderseitigen Gummibandbundbereich (84), einen rückseitigen Gummibandbundbereich (86), einen Schrittbereich (30), eine Taillenöffnung und zwei Beinöffnungen; der Artikel (20) ferner umfassend eine mittlere Grundeinheit (38), umfassend einen Absorptionskern (62), der sich in Längsrichtung von dem vorderseitigen Gummibandbundbereich (84) zu dem rückseitigen Gummibandbundbereich (86) erstreckt;

wobei die mittlere Grundeinheit (38) einen Absorptionskern (62) umfasst, umfassend eine Oberschicht, eine Unterschicht und einen Absorptionskern (62), der zwischen der Oberschicht und der Unterschicht angeordnet ist, der Absorptionskern (62) ferner umfassend eine Absorptionsschicht und eine Aufnahmeschicht, wobei die Absorptionsschicht im Wesentlichen cellulosefrei ist,
wobei der vorderseitige Rand der Absorptionsschicht von dem vorderseitigen Rand des Aufnahmeschichtrands entfernt gelegen eingerichtet ist, wobei der vorderseitige Rand der Aufnahmeschicht der vorderseitige Absorptionskernsteifigkeitsrand (52SE) ist;
wobei der vorderseitige und der rückseitige Gummibandbundbereich (84, 86) jeweils querverlaufende ununterbrochene nahe gelegene und entfernt gelegene Ränder aufweist, wobei sich der nahe gelegene Rand (90) näher als der entfernt gelegenen Rand (88), in Bezug auf die längsgerichtete Mitte des Artikels, befindet;
wobei der vorderseitige Gummibandbundbereich (84) den vorderseitigen Absorptionskernsteifigkeitsrand (52SE) aufweist, wobei der Abstand von dem entfernt gelegenen Rand (88) zu dem vorderseitigen Absorptionskernsteifigkeitsrand (52SE) eine Länge L2 definiert, wobei L2 nicht größer als 77 mm ist, und wobei der vorderseitige Gummibandbundbereich (84) in 3 Bereiche unterteilt ist, die sich in Querrichtung erstrecken und der durch seinen Position von dem entfernt gelegenen Rand (88) definiert ist, wobei 0-50 % von L2 ein Bereich 1 sind, 50-100 % von L2 ein Bereich 2 sind und der Rest des vorderseitigen Gummibandbundbereichs (84) ein Bereich 3 ist;
wobei der Bereich 1 eine vertikale Steifigkeit von Z1 aufweist, der Bereich 2 eine vertikale Steifigkeit von Z2 aufweist und der Bereich 3 eine vertikale Steifigkeit von Z3 aufweist, nach den Messungen hierin, und eine Beziehung von Z1 ≤ Z2 ≤ Z3 aufweisen;
wobei der Artikel (20) eine Dehnungsumfangskraft und einen vollen Umfang nach den Messungen hierin aufweist, wobei das Verhältnis der Dehnungsumfangskraft zu dem vollen Umfang weniger als 0,01 (N/mm) beträgt,
wobei der Artikel (20) eine Dehnungsumfangskraft von weniger als 7 N nach den Messungen hierin aufweist, und wobei der Artikel (20) eine Passumfangskraft von mehr als 2 N nach den Messungen hierin aufweist, und
wobei der vorderseitige Gummibandbundbereich (84) und der rückseitige Gummibandbundbereich (86) einen ringartigen Gummibandbund (40) ausbilden, wobei die Mitte des vorderseitigen Gummibandbundbereichs (84) mit einem vorderseitigen Taillenfeld (52) der mittleren Grundeinheit (38) gefaltet ist, die Mitte des rückseitigen Gummibandbundbereichs (86) mit einem rückseitigen Taillenfeld (54) der mittleren Grundeinheit (38) gefaltet ist, der vorderseitige und der rückseitige Gummibandbundbereich (84, 86) jeweils ein linkes Seitenfeld und ein rechtes Seitenfeld (82) aufweisen, in dem die mittlere Grundeinheit (38) nicht überlappt, wobei der vorderseitige und der rückseitige Gummibandbundbereich (84,86) jeweils ein Laminat einer inneren Lage (94), einer äußeren Lage (92) und einer Vielzahl von Gummibandsträngen sind, die in Sandwichform dazwischen angeordnet sind, wobei alle der Gummibandstränge, die zwischen der inneren Lage (94) und der äußeren Lage (92) in Sandwichform angeordnet sind, in Querrichtung im Wesentlichen parallel zueinander verlaufen, die Gesamtheit der Länge des Bundseitenrands des vorderseitigen Gummibandbundbereichs (84) mit einer gewissen Länge des Bundseitenrands des rückseitigen Gummibandbundbereichs (86) genäht ist, um eine Nahtlänge LS zu definieren;

wobei der rückseitige Gummibandbundbereich (86) in 3 Bereiche unterteilt ist, die sich in Querrichtung erstrecken, und durch seine Position von dem entfernt gelegenen Rand (88) zu dem nahe gelegenen Rand (90) bezüglich des Prozentsatzes der Nahtlänge LS definiert ist, wobei 0-30 % eine Taillenbereich (102B) ist, 30-75 % eine Bauchbereich (105B) ist und 75-100 % eine Beinbereich (108B) ist, wobei die Zugspannungsbeanspruchung des Bauchbereichs (105B) höher als die Zugspannungsbeanspruchung eines beliebigen anderen Bereichs ist.

2. Artikel (20) nach Anspruch 1, wobei Z3 weniger als 65 gf nach den Messungen hierin beträgt.

3. Artikel (20) nach einem der vorstehenden Ansprüche, wobei die Zugspannungsbeanspruchung von Bereich 3 höher als die Zugspannungsbeanspruchung von einem beliebigen Bereich 1 oder 2 ist.

4. Artikel (20) nach einem der vorstehenden Ansprüche, wobei die Absorptionsschicht einen sich in Längsrichtung erstreckenden Kanal umfasst, wobei der Kanal im Wesentlichen frei von Absorptionsmaterial ist.

5. Artikel (20) nach einem der vorstehenden Ansprüche, wobei ein Kanal ferner in der Aufnahmeschicht ausgebildet ist, wobei der Kanal in der Aufnahmeschicht wenigstens teilweise mit dem Kanal der Absorptionsschicht in der Dickenrichtung übereinstimmt.

## Revendications

1. Article (20) absorbant continu dans une direction longitudinale et une direction transversale comprenant une région de ceinture élastique avant (84), une région de ceinture élastique arrière (86), une région d'entrejambe (30), une ouverture de taille et deux ouvertures de jambe ; l'article (20) comprenant en outre un châssis central (38) comprenant une âme absorbante (62) qui s'étend longitudinalement de la région de ceinture élastique avant (84) à la région de ceinture élastique arrière (86) ;

dans lequel le châssis central (38) comprend une âme absorbante (62) comprenant une feuille de dessus, une feuille de fond et une âme absorbante (62) positionnée entre la feuille de dessus et la feuille de fond, l'âme absorbante (62) comprenant en outre une couche absorbante et une couche de recueil, dans lequel la couche absorbante est sensiblement exempte de cellulose,

dans lequel le bord avant de la couche absorbante est disposé distal par rapport au bord avant du bord de couche de recueil, dans lequel le bord avant de la couche de recueil est le bord de rigidité d'âme absorbante avant (52SE) ;

les régions de ceinture élastique avant et arrière (84, 86) ayant chacune des bords proximal et distal transversalement continus, le bord proximal (90) étant localisé plus près que le bord distal (88) par rapport au centre longitudinal de l'article ;

la région de ceinture élastique avant (84) ayant le bord de rigidité d'âme absorbante avant (52SE) avec la distance allant du bord distal (88) au bord de rigidité d'âme absorbante avant (52SE) définit une longueur L2, dans lequel L2 n'est pas supérieure à environ 77 mm, et dans lequel la région de ceinture élastique avant (84) est divisée en 3 zones s'étendant dans la direction transversale et définie par sa localisation par rapport au bord distal (88) dans lequel ; 0 à 50 % de L2 est une Zone 1, 50 à 100 % de L2 est une Zone 2, et le reste de la région de ceinture élastique avant (84) est une Zone 3 ;

dans lequel la Zone 1 a une rigidité verticale de Z1, la Zone 2 a une rigidité verticale de Z2 et la Zone 3 a une rigidité verticale de Z3 selon les mesures ici, et ont une relation de $Z1 \leq Z2 \leq Z3$ ;

dans lequel l'article (20) a une force circonférentielle d'étirement et une circonférence complète selon les mesures ici, dans lequel le rapport de la force circonférentielle d'étirement à la circonférence complète est inférieur à 0,01 (N/mm).

dans lequel l'article (20) a une force circonférentielle d'étirement inférieure à 7 N selon les mesures ici, et dans lequel l'article (20) a une force circonférentielle d'ajustement de plus de 2 N selon les mesures ici, et dans lequel la région de ceinture élastique avant (84) et la région de ceinture élastique arrière (86) forment une ceinture élastique de type anneau (40), le centre de la région de ceinture élastique avant (84) est joint à un pan de taille avant (52) du châssis central (38), le centre de la région de ceinture élastique arrière (86) est joint à un pan de taille arrière (54) du châssis central (38), les régions de ceinture élastique avant et arrière (84, 86) ayant chacune un pan latéral gauche et un pan latéral droit (82) où le châssis central (38) ne chevauche pas, dans lequel les régions élastiques avant et arrière (84, 86) sont chacune un stratifié d'une feuille interne (94), d'une feuille externe (92) et d'une pluralité de fils élastiques placés en sandwich entre elles, dans lequel la totalité des fils élastiques placés en sandwich entre la feuille interne (94) et la feuille externe (92) s'étendent dans la direction transversale sensiblement parallèles les uns aux autres, l'entièreté de la longueur du bord latéral de ceinture de la région de ceinture élastique avant (84) est jointe avec une certaine longueur du bord latéral de ceinture de la région de ceinture élastique arrière (86) pour définir une longueur de jointure LS ;

dans lequel la région de ceinture élastique arrière (86) est divisée en 3 zones s'étendant dans la direction transversale et est définie par sa localisation allant du bord distal (88) au bord proximal (90) par rapport au pourcentage de la longueur de jointure LS dans lequel ; 0 à 30 % est une zone de taille (102B), 30 à 75 % est une zone ventrale (105B) et 75 à 100 % est une zone de jambe (108B), dans lequel la contrainte de traction de la zone ventrale (105B) arrière est supérieure à la contrainte de traction de l'une quelconque autre zone.

2. Article (20) selon la revendication 1 dans lequel Z3 est inférieure à 65 gf selon les mesures ici.

3. Article (20) selon l'une quelconque des revendications précédentes dans lequel la contrainte de traction de la Zone 3 est supérieure à la contrainte de traction de l'une quelconque des Zones 1 ou 2.

4. Article (20) selon l'une quelconque des revendications précédentes dans lequel la couche absorbante comprend un canal s'étendant longitudinalement, le canal étant sensiblement dépourvu de matériau absorbant.

5. Article (20) selon l'une quelconque des revendications précédentes dans lequel un canal est en outre formé dans la couche de recueil, dans lequel le canal dans la couche de recueil concorde au moins partiellement avec le canal de la couche absorbante dans la direction d'épaisseur.

FIG. 1

FIG. 2

FIG. 3

FIG. 4A

FIG. 4B

FIG. 4C

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200617718 A
- WO 2016029370 A
- EP 2517681 A
- WO 2016101195 A
- WO 2011087503 A